# EUROPEAN PATENT APPLICATION

(11) **EP 3 121 289 A1**
(43) Date of publication of application: **25.01.2017**
(21) Application number: 15177865.1
(22) Date of filing: 22.07.2015
(51) Int. Cl.: C12Q 1/68

(54) **MICRORNAS AS BIOMARKERS OF BILE DUCT DISEASES**

(71) Applicant: Humanitas Mirasole S.p.A., 20100 Rozzano (MI) (IT)
(72) Inventor: Invernizzi, Pietro, 20089 Rozzano (IT); Bernuzzi, Francesca, Veronica, 20089 Rozzano (IT); Lleo de Nalda, Ana, 20089 Rozzano (IT)
(74) Representative: Capasso, Olga

(57) **Abstract**

The present invention refers to an in vitro method for diagnosing and/or for assessing the risk and/or for prognosing and/or for monitoring the progression and/or for monitoring the efficacy of a therapeutic treatment and/or for the screening of a therapeutic treatment of a disease of the bile ducts in a subject comprising the steps of detecting at least one microRNA selected from the group consisting of: miR-483-5p, miR-194, miR-222 and miR-200c in an isolated biological sample, wherein the disease of the bile ducts is selected from the group consisting of: Cholangiocarcinoma (CCA) and Primary sclerosing cholangitis (PSC).

SEQUENCE LISTING

<110>  Humanitas Mirasole S.p.A.

<120>  MICRORNAS AS BIOMARKERS OF BILE DUCT DISEASES

<130>  BE 126457

<160>  7

<170>  PatentIn version 3.5

<210>  1
<211>  22
<212>  RNA
<213>  Homo sapiens

<400>  1
aagacgggag gaaagaaggg ag                                                22

<210>  2
<211>  22
<212>  RNA
<213>  Homo sapiens

<400>  2
uguaacagca acuccaugug ga                                                22

<210>  3
<211>  21
<212>  RNA
<213>  Homo sapiens

<400>  3
agcuacaucu ggcuacuggg u                                                 21

<210>  4
<211>  23
<212>  RNA
<213>  Homo sapiens

<400>  4
uaauacugcc ggguaaugau gga                                               23

<210>  5
<211>  22
<212>  RNA
<213>  Homo sapiens

<400>  5
aacuggcccu caaagucccg cu                                                22

<210>  6
<211>  22
<212>  RNA
<213>  Homo sapiens

<400>  6

uggaguguga caaugguguu ug                                                22

<210>  7
<211>  22
<212>  RNA
<213>  Homo sapiens

<400>  7
uccauuacac uacccugccu cu                                                22

## Description

### Field of the invention

The present invention relates to an in vitro method for assessing the risk and/or for diagnosing and/or for prognosing and/or for monitoring the progression and/or for monitoring the efficacy of a therapeutic treatment and/or for the screening of a therapeutic treatment of a disease of the bile ducts in a subject comprising the detection of at least one microRNA selected from the group consisting of: miR-483-5p, miR-194, miR-222 and miR-200c in an isolated biological sample obtained from the subject, wherein the disease of the bile ducts is selected from the group consisting of: Cholangiocarcinoma (CCA) and Primary sclerosing cholangitis (PSC).

### Background art

Primary sclerosing cholangitis (PSC) is a chronic cholestatic syndrome of unknown etiology characterized by fibrosing inflammatory destruction of the intra- and extrahepatic bile ducts. It is frequently progressive and may be fatal in the absence of liver transplantation. The incidence and prevalence rates for PSC range from 0 to 1.3 per 100.000 inhabitants/year and 0-16.2 per 100.000 inhabitants, respectively. (Boonstra K. et al., Journal of Hepatology 2012 vol. 56 j 1181-1188).

Death from cancer occurs in a significant subset of patients with PSC and Cholangiocarcinoma (CCA) is the cancer most frequently associated with PSC. The risk of CCA was 160-fold greater than in the general population, and 37% of CCAs were diagnosed less than 1 year after the diagnosis of PSC.

CCA may occur in the absence of advanced hepatic fibrosis and many patients are diagnosed with CCA early in their fourth decade of life.

PSC is the commonest known predisposing factor for CCA in the West. CCA rates of up to 40% have been reported in PSC patients. In East Asia, where the disease is common, CCA has been pathogenically associated with liver fluke infestation, particularly the endemic Opisthorcis viverrini. Up to 10% of patients with intrahepatic biliary stones develop CCA. Cirrhosis from any cause is associated with an increased risk of CCA. (Ghouri Y.A. et al., J Carcinog. 2015; 14: 1)

Early diagnosis of PSC and CCA, also in the setting of PSC, is the only approach for curative outcomes, but this is difficult due to the lack of sensitive and specific biomarkers (Yimam KK et al, Autoimmune Rev. 2014; 13:445-50) (Gatto M. et al., World J Gastrointest Oncol 2010: 2:136-45).

WO2012120374 relates to methods and biomarkers (e.g., epigenetic biomarkers) for detection of gastrointestinal cancers (e.g., colorectal cancer, gastric cancer, pancreatic cancer, liver cancer, cancer of the gall bladder and/or bile ducts (e.g., cholangiocarcinoma)) in biological samples (e.g., tissue samples, stool samples, blood samples, plasma samples, cell samples, gall samples, bile samples, serum samples). In particular the disclosed method comprises determining the level, presence, or frequency of methylation of specific nucleic acid polymers. WO9202819 refers to a method for detecting the presence of diseases including ulcerative colitis or primary sclerosing cholangitis in a patient. The method consists of contacting a body sample of the patient with immobilized neutrophils so as to allow the antineutrophilic cytoplasmic antibodies to bind to the neutrophils. Further, the method provides detecting bound anti-neutrophilic cytoplasmic antibodies, the presence of the antibodies indicative of the disease state.

However, the discovery of non-invasive specific biomarkers that can be routinely measured in easily accessible samples, contributing to establish a diagnosis of PSC or of CCA is an unmet clinical need. In fact, to date such diagnoses are not only difficult to achieve, but may remain uncertain even after imaging and cytological sampling have been attempted.

Specific serum microRNAs (miRNAs) have been reported in a number of inflammatory and neoplastic conditions (Gilad S, et al., PLoS One 2008: e3148).

Cell-free miRNAs in body fluids are stable under harsh conditions including boiling, low/high pH, extended storage and multiple freeze-thaw cycles (Chen X et al.; Cell Res 2008; Mitchell PS et al.; Proc Natl Acad Sci U S A 2008; Gilad S. et al.; PLoS One 2008). Recently, advances in studies of circulating miRNAs have led to the concept that tissue or organ specific intracellular miRNAs may be released into the circulation during cell death or apoptosis owing to cell turnover, cellular destruction or pathological injury.

Therefore, miRNA profiles in serum may potentially be used as biomarkers that could improve diagnosis and the clinical management of diseases.

To date there are no data on miRNAs in PSC. MiRNA expression in CCA have been reported only in small cohorts, perhaps due to the rarity of CCA (Brase JC.; Mol Cancer 2010).

It has been found that miRNAs are involved in the pathogenesis of CCA, as specific miRNAs are either upregulated (Meng F.; Gastroenterology 2006), or downregulated (Meng F.; Oncogene 2008, Mott JL.; Oncogene 2007). miRNAs in human bile has been investigated as CCA biomarkers, however, bile samples were obtained by endoscopic retrograde cholangiopancreatography, an invasive procedure with associated complications.

Therefore there is still the need to identify a panel of deregulated miRNAs in serum samples from PSC and CCA patients and investigate their potential as biomarkers for detection and staging of disease.

### Description of the invention

In the present invention it was surprisingly found that specific patterns of pathology-specific deregulated miRNA exist that may be used as a signature of PSC and CCA and that are therefore able to support each specific diagnosis.

In this study by profiling the serum miRNA expression in PSC and CCA inventors showed that a number of miRNAs are deregulated. In particular inventors have identified: 1) a PSC-associated serum miRNA (miR-200c) that can supplement the current diagnosis of this disease; 2) a group of serum miRNAs (miR-483-5p and/or miR-194 and/or miR-222) associated to CCA that can be used for the diagnosis of this cancer; 3) a group of serum miRNAs (miR-222 and/or miR-483-5p) that characterize the evolution of PSC to CCA and therefore might allow a very early diagnosis in individuals at risk of developing this dangerous cancer; and finally, a group of serum miRNAs altered in all diseases studied, that can be considered non-specific. Importantly, inventors herein identify PSC- and CCA-associated miRNAs as potential biomarkers.

Indeed miR-200c, may be used for diagnostic purposes since there are no specific PSC markers available.

Similarly, miR-194, miR-483-5p and miR-222 can allow the diagnosis of CCA. Particular attention should be paid to miR-222, and miR-483-5p both upregulated in CCA compared to PSC. The combined use of miR-222 and miR-483-5p could be used to monitor the course of PSC patients for early CCA detection.

Very important is the validation as biomarkers of miR-483-5p, upregulated in CCA compared to CTR and PSC. In contrast, miR-200c was downregulated in PSC compared to CTR and then upregulated in CCA compared to PSC.

Taken together, the identified miRs allow to diagnose and monitor PSC and CCA patients, and may be used to identify an early development of the tumor.

Object of the invention is an in vitro method for diagnosing and/or assessing the risk of developing and/or prognosing and/or for monitoring the progression and/or for monitoring the efficacy of a therapeutic treatment and/or for the screening of a therapeutic treatment of a disease of the bile ducts in a subject comprising the steps of:
a) detecting at least one microRNA selected from the group consisting of:
   miR-483-5p, miR-194, miR-222 and miR-200c
   in an isolated biological sample obtained from the subject and
b) comparing with respect to a proper control
   wherein the disease of the bile ducts is selected from the group consisting of:
   Cholangiocarcinoma (CCA) and Primary sclerosing cholangitis (PSC).

In a preferred embodiment, the in vitro method for diagnosing and/or assessing the risk of developing and/or prognosing CCA as above defined, comprises the steps of:
a) measuring the amount of miR-483-5p and/or miR-194 and/or miR-222 in said isolated biological sample obtained from the subject and
b) comparing the measured amount of step a) with a proper control amount,
wherein an amount of said miR-483-5p and/or miR-194 and/or miR-222 in the isolated biological sample obtained from the subject higher than the control amount indicates that the subject is either affected by or is at increased risk for developing CCA.

In a preferred embodiment, the in vitro method for diagnosing and/or assessing the risk of developing and/or prognosing Cholangiocarcinoma (CCA) according to the invention, comprises the steps of:
a) measuring the amount of miR-483-5p and/or miR-194
   in said isolated biological sample obtained from the subject and
b) comparing the measured amount of step a) with a proper control amount,
wherein an amount of said miR-483-5p and/or miR-194 in the isolated biological sample obtained from the subject higher than the control amount indicates that the subject is either affected by or is at increased risk for developing CCA.

In an alternative preferred embodiment, the in vitro method for diagnosing and/or assessing the risk of developing and/or prognosing Primary sclerosing cholangitis (PSC) as above defined, comprises the steps of:
a) measuring the amount of miR-200c
   in said isolated biological sample obtained from the subject and
b) comparing the measured amount of step a) with a proper control amount,
wherein an amount of said microRNA in the isolated biological sample obtained from the

subject lower than the control amount indicates that the subject is either affected by or is at increased risk for developing PSC.

In a further preferred embodiment, the in vitro method for diagnosing and/or assessing the risk of developing and/or prognosing CCA as above defined, comprises the steps of:
a) measuring the amount of miR-222 and/or miR-483-5p and/or miR-194
   in said isolated biological sample obtained from the subject and
b) comparing the measured amount of step a) with a proper control amount from a PSC patient, wherein an amount of said miR-222 and/or miR-483-5p and/or miR-194 in the isolated biological sample obtained from the subject higher than the control amount indicates that the subject is either affected by or is at increased risk for developing CCA.

In preferred embodiment, the in vitro method for diagnosing and/or assessing the risk of developing and/or prognosing CCA according to the invention, comprises the steps of:
a) measuring the amount of miR-222 and/or miR-483-5p
   in said isolated biological sample obtained from the subject and
b) comparing the measured amount of step a) with a proper control amount from a PSC patient, wherein an amount of said miR-222 and/or miR-483-5p in the isolated biological sample obtained from the subject higher than the control amount indicates that the subject is either affected by or is at increased risk for developing CCA.

Preferably, the in vitro method for monitoring the progression and/or for monitoring the efficacy of a therapeutic treatment and/or for the screening of a therapeutic treatment of CCA or PSC as above defined, comprises the steps of:
a) measuring the alteration of the amount of at least one microRNA selected from the group consisting of:
   miR-483-5p, miR-194, miR-222 and miR-200c
   in said isolated biological sample obtained from the subject and
b) comparing the measured amount of step a) with a proper control amount.

In a preferred embodiment of the methods of the invention, the microRNAs to be detected or measured in step a) are:
- at least miR-483-5p and miR-194, or
- at least miR-222 and miR-483-5p, or
- at least miR-222 and miR-194, or
- at least miR-222, miR-483-5p and miR-194, or
- at least miR-200c.

Preferably, the biological sample is a blood sample, more preferably a serum sample, a whole blood sample, or a plasma sample.

The method according to the invention preferably further comprises the step of extracting RNA from the biological sample.

Preferably, the detection or the measure of the amount or of the alteration of the amount of the microRNA comprises specific acid nucleic amplification and/or hybridization, e.g. by RT-qPCR.

A further object of the invention is a kit for carrying out the above methods, comprising
- means to detect and/or measure the amount of the at least one microRNA as above defined and optionally,
- control means.

Another object of the invention is a kit to detect and/or measure the amount of the at least one microRNA as above defined consisting of:
- for each of said microRNA, sequence specific amplification means;
- quantitative detection means of said amplified nucleic acids;
- appropriate reagents.

A further object of the invention is a device for measuring the amount of at least one miRNA in a biological sample, wherein said device consists of:
- one or more probe sets for the miRNA as above defined and
- solid supporting means.

Preferably said device is a microarray chip, a QPCR Microfluidic Card, QPCR tubes, QPCR tubes in a strip, or a QPCR plate.

In the present invention, the subject diagnosed with PSC or CCA by the above methods, may be treated with an appropriate therapy. Patients diagnosed with PSC may be treated e.g. with pharmaceutical treatments, e.g. with ursodiol, bile acid sequestrants (cholestyramine), antibiotics and vitamin supplements, or e.g. they may be treated by surgery, e.g. by ERCP, which may involve stenting of the common bile duct, or by liver transplantation. Patients diagnosed with CCA may be treated with surgical resection or liver transplantation.

In the present invention, any combination (e.g. of two, three, four) of the above defined miRs may be detected or their amount or alteration measured.

In the case of a method or a kit for diagnosing and/or assessing the risk of developing and/or prognosing CCA or PSC, the proper control may be a sample taken from a healthy patient or from a patient affected by another disorder or pathology, and the proper control amount may be the amount of the same microRNA(s) measured in a sample taken from a healthy patient or from a patient affected by another disorder or pathology.

In the case of a method or a kit for monitoring the progression of the CCA or PSC, the progress of the disease is monitored and the proper control may be a sample taken from the same subject at various times or from another patient, and the proper control amount may by the amount of the same microRNA(s) measured in a sample taken from the same subject at various times or from another patient.

If by comparing the measured amount of miR-200c with the amount obtained from a control sample, the amount of said microRNA in the sample isolated from the subject corresponds to a lower value, the subject may present PSC, may be at risk of developing PSC or go towards an aggravation of said disease.

If by comparing the measured amount of miR-200c with the amount obtained from a control sample, the amount of said microRNA in the sample isolated from the subject corresponds to a similar or higher value, the subject may be not affected by PSC, may not be at risk of developing PSC or go toward an amelioration of the disease, respectively.

If by comparing the measured amount of miR-483-5p and/or miR-194 and/or miR-222 with the amount obtained from a control sample, the amount of said microRNA(s) in the sample isolated from the subject correspond(s) to a higher value, the subject may present CCA, may be at risk of developing CCA or go towards an aggravation of said disease.

If by comparing the measured amount of the miR-483-5p and/or miR-194 and/or miR-222 with the amount obtained from a control sample, the amount of said microRNA in the sample isolated from the subject corresponds to a similar or lower value, the subject may be not affected by CCA, may not be at risk of developing CCA or go toward an amelioration of the disease, respectively.

In the case of a method or a kit for monitoring the efficacy of a therapeutic treatment, the proper control may by a sample taken from the same subject before initiation of the therapy or taken at various times during the course of the therapy and the proper control amount may be the amount of the same microRNA(s) measured in a sample taken from the same subject before initiation of the therapy or taken at various times during the course of the therapy. In this case, if the amount of miR-200c in the isolated biological sample obtained from the subject is higher than the control amount, it may indicate that the therapeutic treatment is effective, or if the amount of miR-483-5p and/or miR-194 and/or miR-222 in the isolated biological sample obtained from the subject is lower than the control amount, it may indicate that the therapeutic treatment is effective.

In the case of a method or a kit for the screening of a therapeutic treatment, the proper control may be a sample taken from subjects without treatment, or from subjects treated with a substance that is to be assayed or from subjects treated with a reference treatment and the proper control amount may be the average of the amounts of the same microRNA measured in samples taken from subjects without treatment, or from subjects treated with a substance that is to be assayed or from subjects treated with a reference treatment. In this case, if the amount of miR-200c in the isolated biological sample obtained from the subject is higher or equal than the control amount, it may indicate that the tested substance is effective for the treatment of PSC, or if the amount of miR-483-5p and/or miR-194 and/or miR-222 in the isolated biological sample obtained from the subject is lower or equal than the control amount, it may indicate that the tested substance is effective for the treatment of CCA.

In the context of the present invention, the term "detecting" may be intended also as "measuring the amount".

In the present invention, the expression "measuring the amount" can be intended as measuring the amount or concentration or level of the respective miRNA and/or DNA thereof, preferably semi-quantitative or quantitative. The term "amount", as used in the description refers but is not limited to the absolute or relative amount (or concentration or expression level) of miRNA and/or DNA thereof, and any other value or parameter associated with the same or which may result from these. Methods of measuring miRNA and DNA in samples are well known in the art. In order to detect and/or measure nucleic acid levels, the cells in the isolated biological sample may be lysed, and the levels of miRNA in the lysates or in RNA purified or semipurified from the lysates can be measured by any kind of methods known to the skilled man. Such methods comprise hybridization assays using detectably labeled DNA or RNA probes (e.g. Northern blotting) and/or nucleic acid amplification, e.g. quantitative or semi-quantitative RT-PCR methodologies using appropriate oligonucleotide primers. The expert in the art knows how to design proper primers. Alternatively, quantitative or semi-quantitative in situ hybridization assays can be carried out using, for example, tissue sections, or unlysed cell suspensions, and detectably labeled ( e.g., fluorescent, or enzyme-labeled) DNA or RNA probes. Additional methods for quantifying miRNA include RNA protection assay (RPA), cDNA and oligonucleotide microarrays, representation difference analysis (RDA), differential display, EST sequence analysis, and serial analysis of gene expression (SAGE).

The methods of the invention can further comprise normalizing the expression levels of miRNA. Normalizing includes, but is not limited to adjusting expression levels of miRNA relative to expression levels of one or more nucleic acid in the isolated biological sample. Although the miRNAs tested are indicated as RNA sequences, it will be understood that, when referring to hybridizations or other assays, corresponding DNA sequences can be used as well. For example, RNA sequences may be reverse transcribed and amplified using the polymerase chain reaction (PCR) in order to facilitate detection. In these cases, it will actually be DNA and not RNA that is directly quantitated. It will also be understood that the complement of the reverse transcribed DNA sequences can be analyzed instead of the sequence itself. In this context, the term "complement" refers to an oligonucleotide that has an exactly complementary sequence, i.e. for each adenine there is a thymine, etc. Although assays may be performed for the miRNAs individually, it is generally preferable to assay several miRNAs or to compare the ratio of two or more of the miRNAs.

Alternatively, the expression "detection" , "measuring the amount" or "measuring the alteration" is intended as measuring the alteration of the molecule. Said alteration can reflect an increase or a decrease in the amount of the microRNAs as above defined. An increase of miR-483-5p and/or miR-194 and/or miR-222 can be correlated to an aggravation of CCA. A decrease of miR-483-5p and/or miR-194 and/or miR-222 can be correlated to an amelioration of the CCA or to recovery of the subject.

A decrease of miR-200c can be correlated to an aggravation of PSC. An increase of miR-200c can be correlated to an amelioration of PSC or to recovery of the subject.

In the kit of the invention "control means" are preferably used to compare the amount of the microRNA to a proper control or proper control amount. The "means to detect and/or measure the amount of the microRNA" are known to the expert of the art, and are preferably at least one detectably labeled DNA or RNA probe specific for the above defined miRs and/or miRNA-specific primers for reverse transcribing or amplifying each of the above disclosed miRs. For example, said means may be specific TaqMan probes.

In the kit of the invention, the "sequence specific amplification means" are known to the expert of the art, and are preferably at least one DNA or RNA primer, e.g. stem-looped RT primers. The design of specific probes or miRNA-specific primers is known to the skilled in the art, and appropriate probes and/or primers can be commercially purchased.

The kit of the invention may further comprise appropriate reagents, as e.g. an enzyme for cDNA preparation (e.g., reverse transcriptase) and/or PCR amplification (e.g., Taq polymerase), and/or a reagent for detecting and/or quantifying miRNA. Additionally, the kit may further comprise a reagent for miRNA isolation from samples and/or one or more normalization control(s). The normalization control(s) can, for example, be provided as one or more separate reagent(s) for spiking samples or reactions. Preferably, the normalization control(s) is/are selected from non-endogenous RNA or miRNA, or a miRNA not expressed in the sample.

The kit of the invention preferably comprises instructions for interpreting the obtained data. According to the invention, the amount of the microRNAs miR-483-5p, miR-194, miR-222, miR-200c is preferably determined by detecting a nucleic acid comprising respectively SEQ ID NO: 1, SEQ ID NO:2, SEQ ID NO: 3, SEQ ID NO: 4, a variant or a fragment thereof. Preferably a sequence comprising one of said sequences is its precursor.

Variant nucleic acid sequences may include nucleic acid sequences that have at least about 80% nucleic acid sequence identity with a nucleic acid sequence disclosed herein. Preferably, a variant nucleic acid sequence will have at least about 80%, 81%, 82%, 83%, 84%, 85%, 86%, 87%, 88%, 89%, 90%, 91%, 92%, 93%, 94%, 95%, 96%, 97%, 98%, 99% nucleic acid sequence identity to a full-length nucleic acid sequence or a fragment of a nucleic acid sequence as disclosed herein.

The term "fragments" comprises nucleic acid sequences which may be truncated at the 5'-terminus or 3'-terminus, or may lack internal residues but which maintain their function. Fragments are preferably 10 nt, 15 nt or 20 nt long.

In the present invention it is referred to miRs, microRNAs, miRNAs or hsa-miRs indifferently. MiRNAs referred in the present invention include the sequences in Table 1 (SEQ ID NOs. 1-4) and homologs and analogs thereof, miRNA precursor molecules, and DNA molecules encoding said miRNAs.

**Table 1**

| | |
|---|---|
| miR-483-5p | AAGACGGGAGGAAAGAAGGGAG (SEQ ID NO:1) |
| miR-194 | UGUAACAGCAACUCCAUGUGGA (SEQ ID NO:2) |
| miR-222 | AGCUACAUCUGGCUACUGGGU (SEQ ID NO:3) |
| miR-200c | UAAUACUGCCGGGUAAUGAUGGA (SEQ ID NO:4) |

Preferably the identity of a homolog to a sequence of SEQ ID NOs 1-4 may be at least 90%, more preferably at least 95% identical.

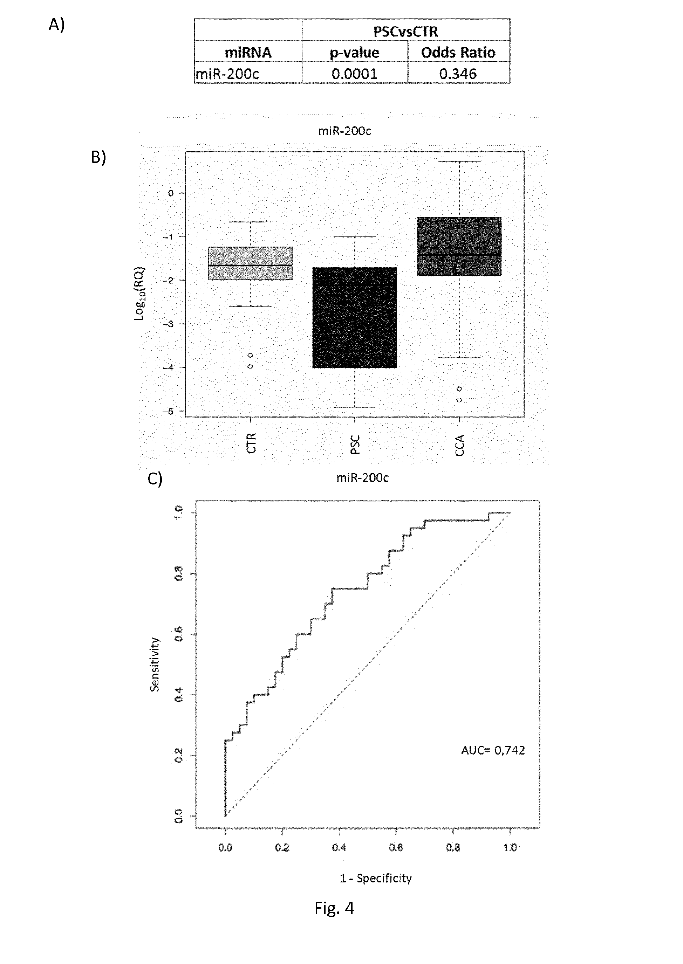

The invention will be now illustrated by means of non-limiting examples referring to the following figures.

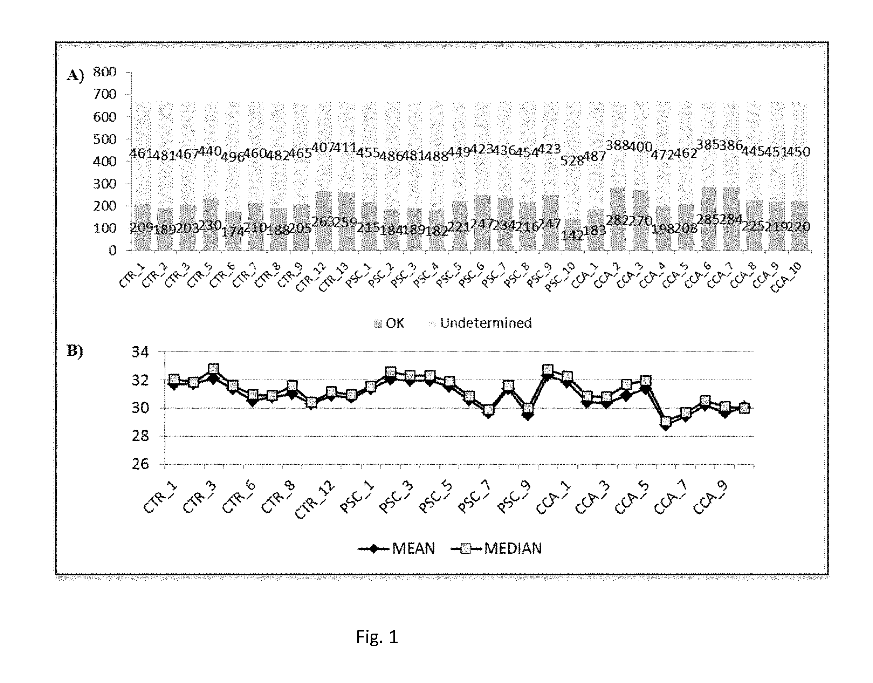

**Figure 1****. miRNA expressed or not expressed from discovery phase** A) miRNAs population consists of more than 200 expressed miRNA for each samples. On average 219 miRNAs detected per sample. B) mean and median of Ct value of each pool before normalization.

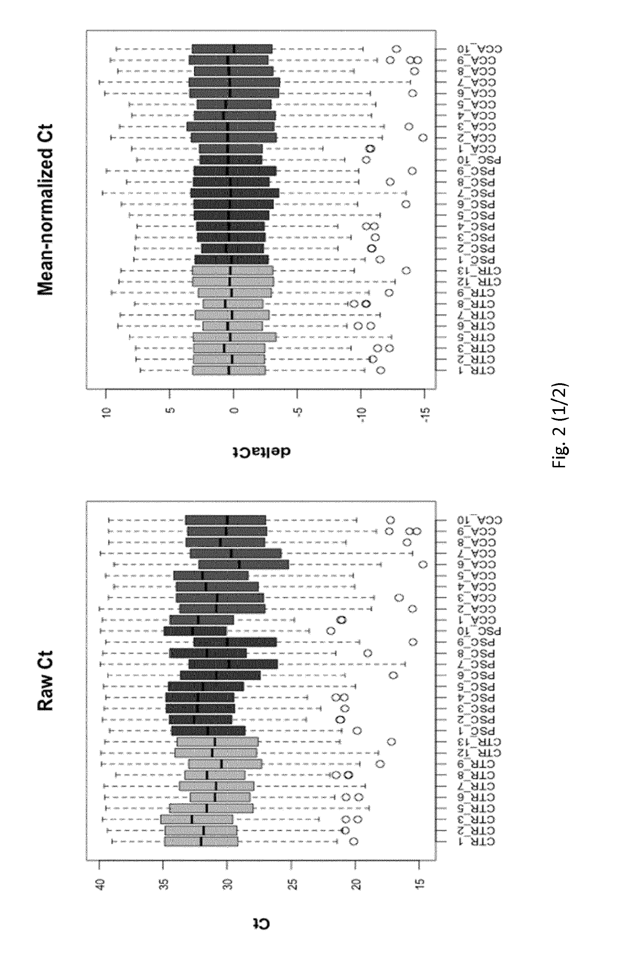

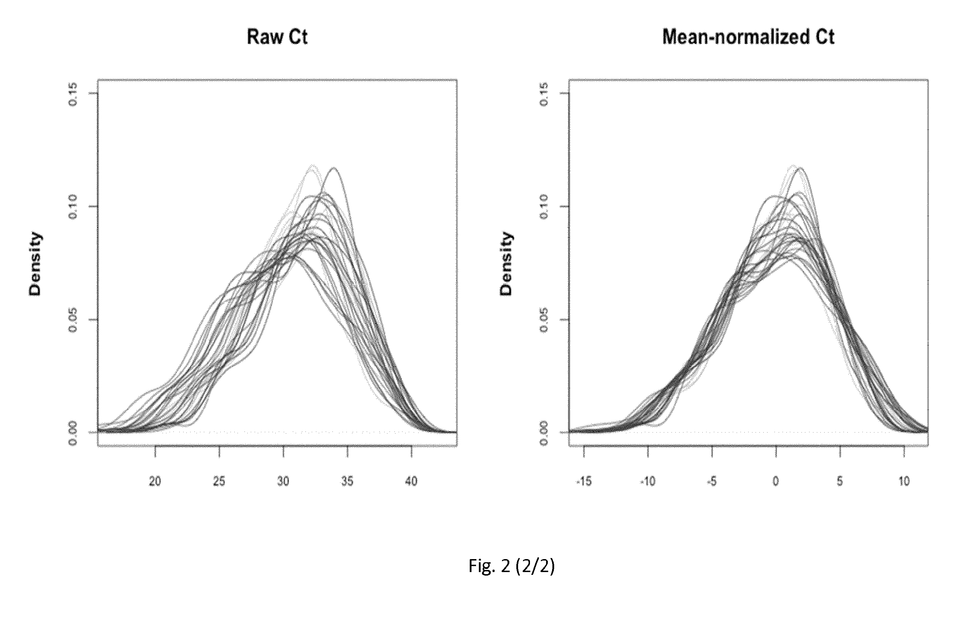

**Figure 2****. Data normalization.** The mean Ct value of all expressed miRNAs (Ct < 40) was used as the normalization factor. The normalized data were properly centered, confirming that part of the technical variability between samples had been removed.

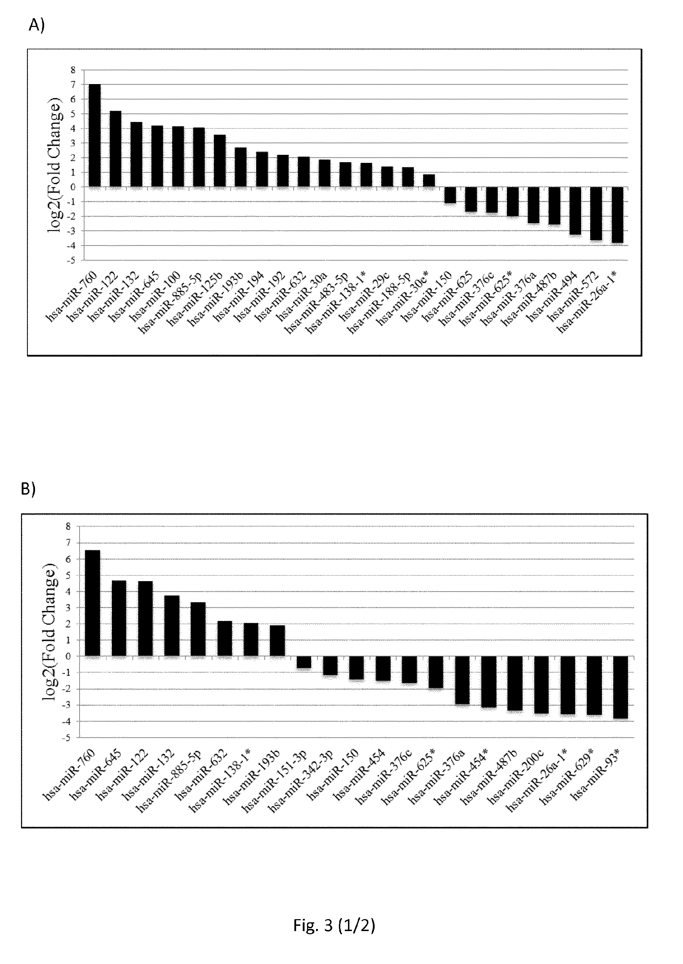

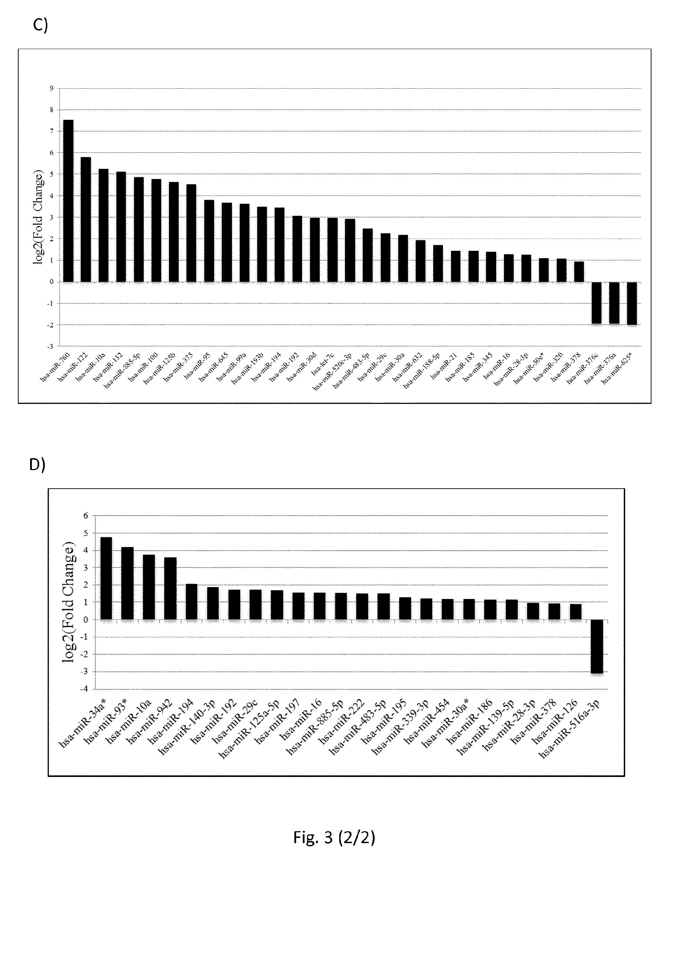

**Figure 3****. Deregulated miRNAs extrapolated from the discovery phase.** A) Deregulated miRNAs in PSC and CCA relative to CTR were identified using a t-test between PSC-CCA and CTR (p<0.05). 183 miRNAs with Ct<40 in >50% samples per group); B) Deregulated miRNAs in PSC relative to CTR (T-test between 10 PSC and 10 CTR (p<0.05). 172 miRNAs with Ct<40 in >50% samples per group); C) Deregulated miRNAs in CCA relative to CTR (T-test between 10 CCA and 10 CTR (p<0.05). 184 miRNAs with Ct<40 in >50% samples per group); D) Deregulated miRNAs in CCA relative to PSC (T-test between 10 CCA and 10 PSC (p<0.05). 177miRNAs with Ct<40 in >50% samples per group).
   miRNAs that did not reach the log2(fold change) >1 are represented with a lighter bar
**Figure 4****. PSC-specific miRNA** A) p-value relative to box plot (B) and Odds Ratio of single miRNA. B) miRNA levels in CTR, PSC, and CCA. Box plots show the minimum, median and maximum of miRNA levels. A standard t-test was performed and a miRNA was considered differently represented if absolute log₁₀(RQ)>1 and p-value <0.05. miR-200c was downregulated in PSC. C) ROC curve analysis of miR-200c. Fraction of true positive results (sensitivity) and false positive results (1-specificity) for levels of miRNA are shown. ROC curves were obtained using logistic regression and the Area Under the Curve (AUC) was calculated using the package epicalc. AUC was considered significant if > 0.70.

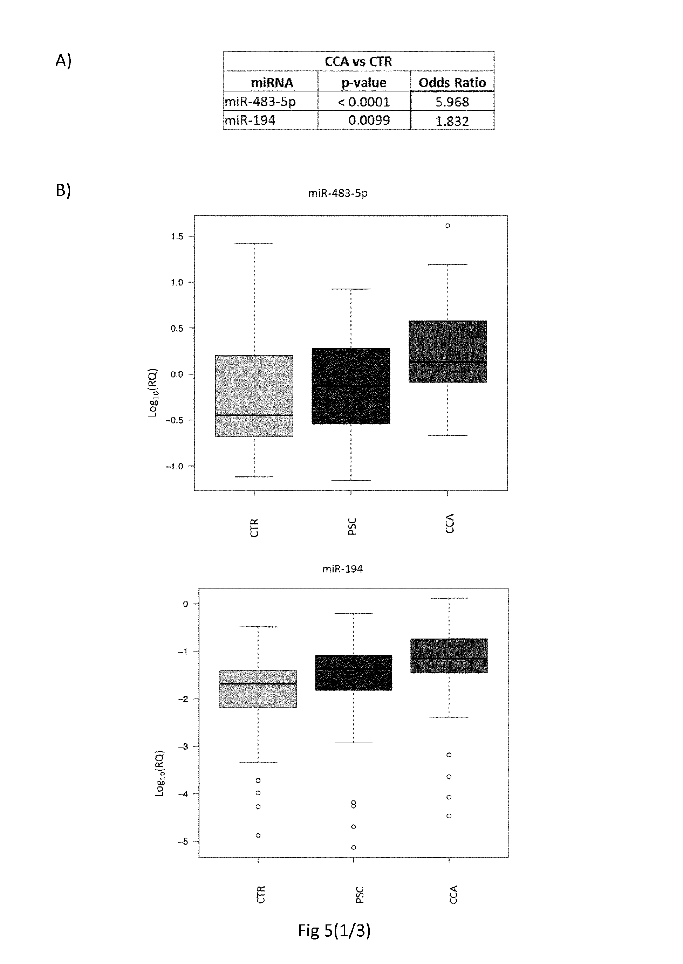

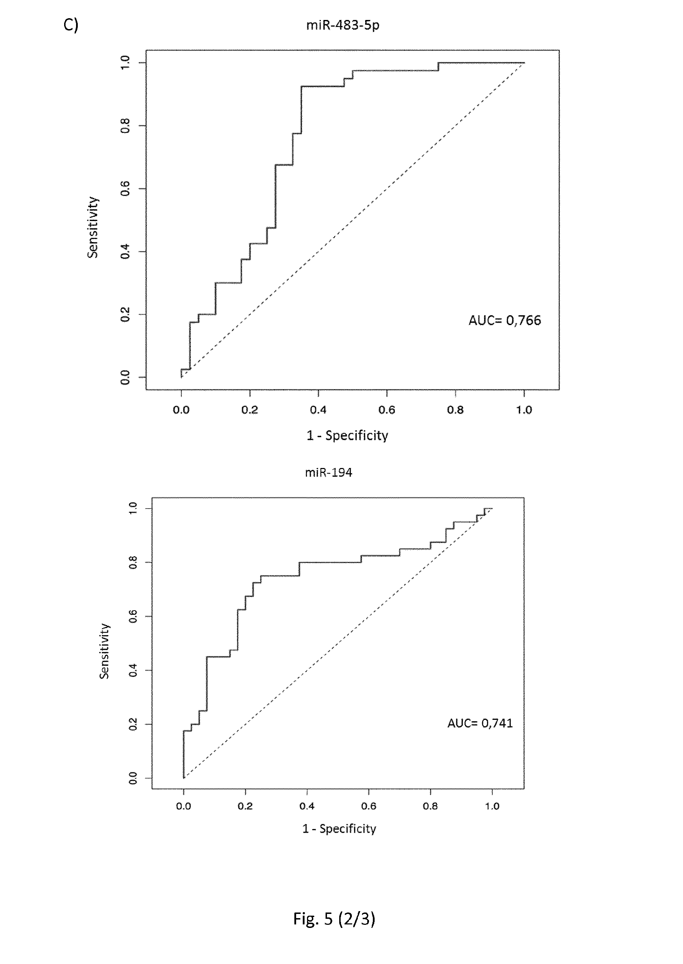

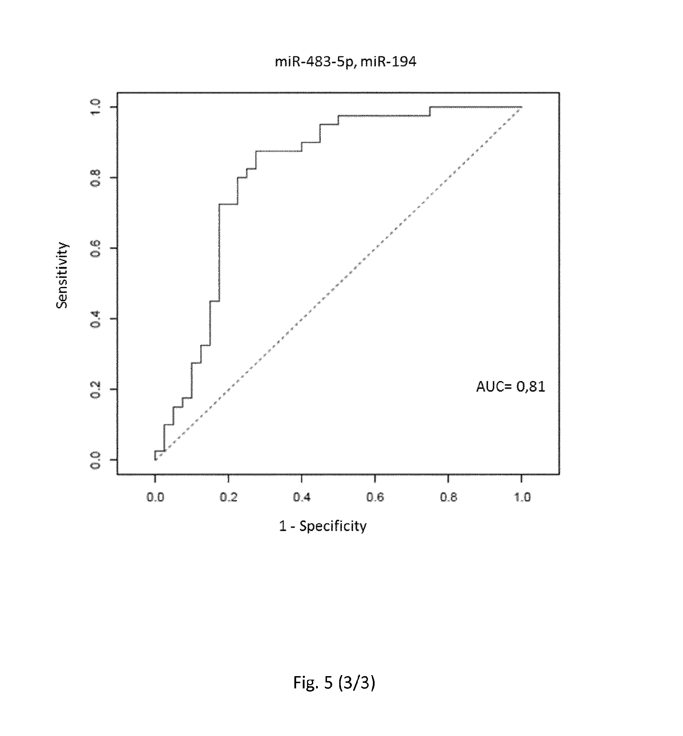

**Figure 5****. CCA- specific miRNAs in CTR** A) p-value relative to box plot (B) and Odds Ratio of single miRNA B) miRNA levels in CTR, PSC, and CCA. Box plots show the minimum, median and maximum for of miRNA levels. A standard t-test was performed and miRNA were considered differently represented if absolute log₁₀(RQ)>1 and p-value <0.05. Two miRNAs were identified: miR-483-5p and miR-194, upregulated in CCA. C) ROC curve analysis of single miRNA and ROC curve analysis combined of the 2 altered miRNA. Fraction of true positive results (sensitivity) and false positive results (1-specificity) for levels of miRNA are shown. ROC curves were obtained using logistic regression and the Area Under the Curve (AUC) was calculated using the package epicalc. AUC was considered significant if > 0.70.

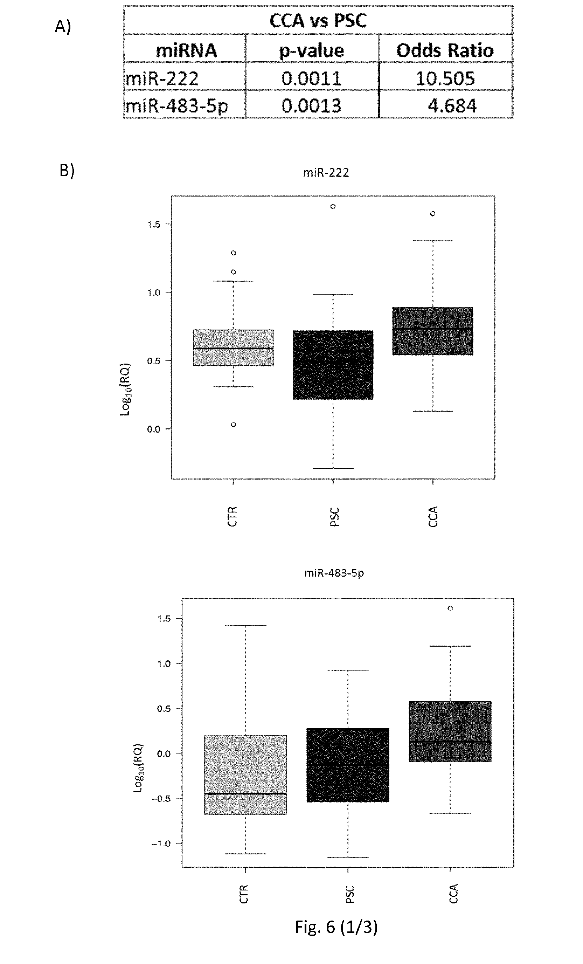

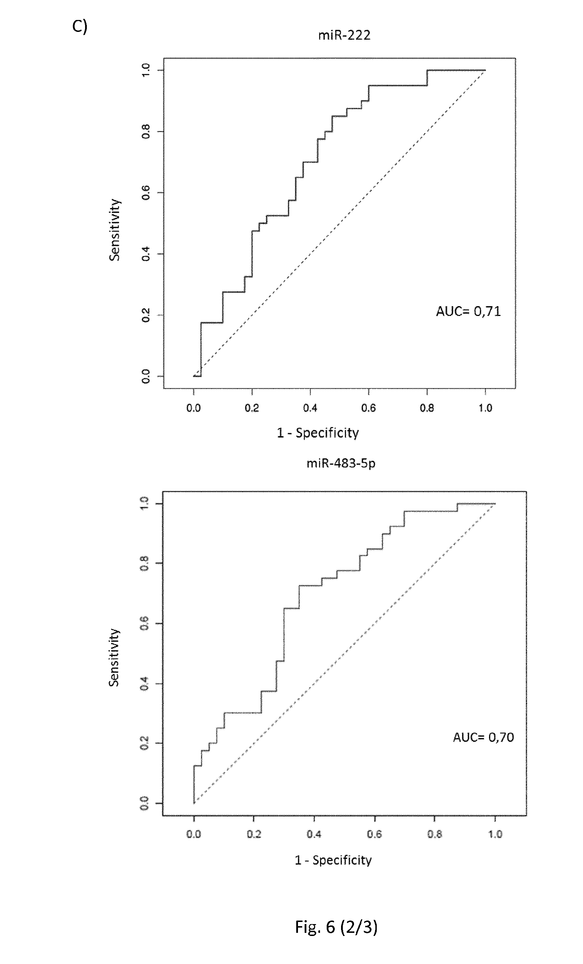

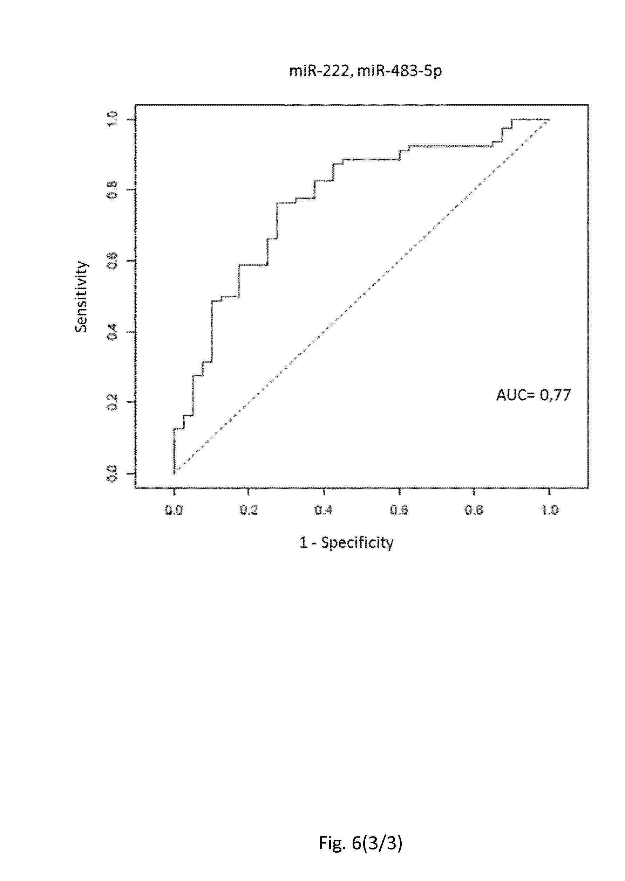

**Figure 6****. CCA- specific miRNAs in PSC** A) p-value relative to box plot (B) and Odds Ratio of CCA-associated miRNA in PSC. B) miRNA levels. The box plot shows the minimum, median and maximum of miRNA level. A standard t-test was performed and miRNA were considered differently represented if absolute log₁₀(RQ)>1 and p-value <0.05 (p-value are showed in the table). Two miRNA were identified: miR-222, and miR-483-5p. C) ROC curve analysis of CCA-associated miRNA and ROC curve analysis combined of the altered miRNAs. Fraction of true positive results (sensitivity) and false positive results (1-specificity) for levels of miRNA are shown. ROC curves were obtained using logistic regression and the Area Under the Curve (AUC) was calculated using the package epicalc. AUC was considered significant if > 0.70.

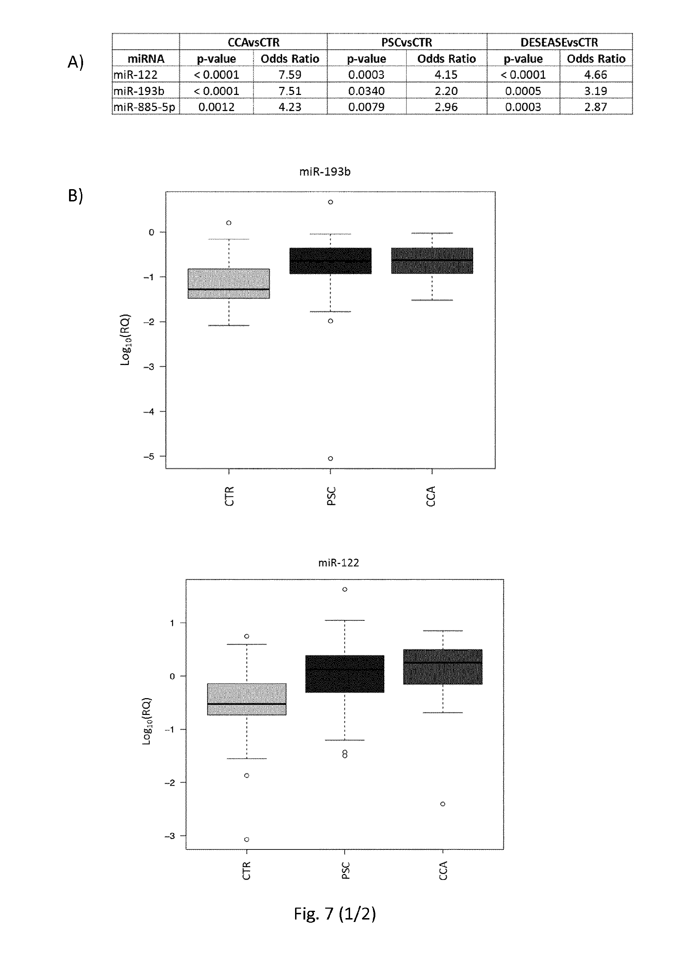

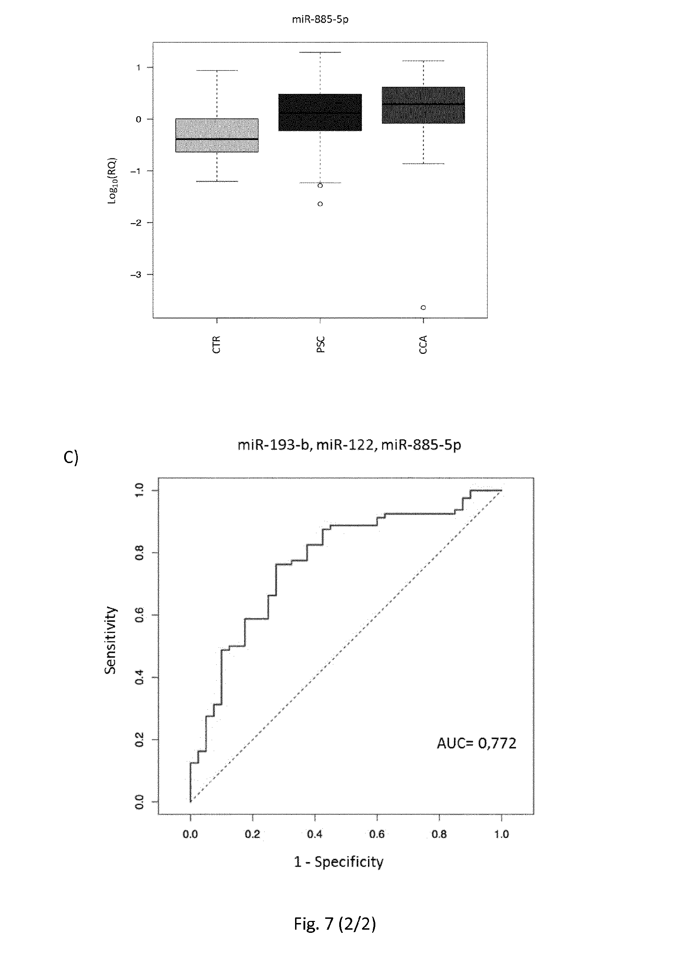

**Figure 7****. Liver disease-correlated miRNAs** A) p-value relative to box plot (B) and Odds Ratio of liver disease specific miRNAs. B) Liver disease specific miRNA levels in CTR, PSC, and CCA. Box plots show the minimum, median and maximum of miRNA levels. A standard t-test was performed and a miRNA was considered differently represented if absolute log₁₀(RQ)>1 and p-value <0.05 (p-value are showed in the table). Three liver disease specific miRNAs were identified: miR-193b, miR-122 and miR-885-5p, all were upregulated in PSC and CCA. C) ROC curve analysis by combination of the 3 miRNAs. Fraction of true positive results (sensitivity) and false positive results (1-specificity) for levels of miRNA are shown. ROC curves were obtained using logistic regression and the Area Under the Curve (AUC) was calculated using the package epicalc. AUC was considered significant if > 0.70.

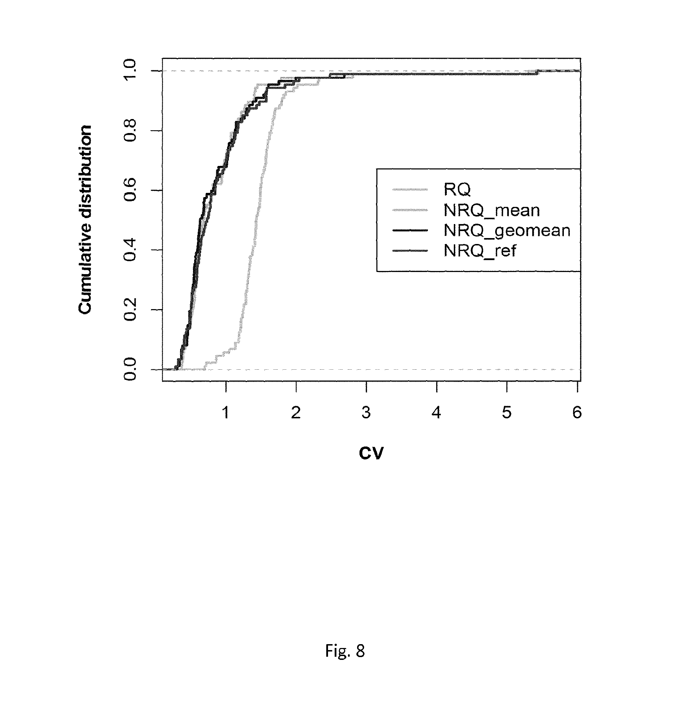

**Figure 8****. Cumulative distribution of coefficients of variation before and after normalization with miR-17, miR-29a, miR-30c and miR-30b.** Cumulative distribution of the coefficient of variation shows the reduction of the variability after normalization. The coefficient of variation (CV) was calculated for miRNAs detected in all samples. Data presented in the plot are either not normalized (RQ), normalized with the arithmetic mean (NRQ_mean), geometric mean (NRQ_geomean) or with four selected reference miRNA (NRQ_ref).

### Materials and Methods

### Human subjects

Patients with CCA (n=70), PSC (n=70), and healthy control (CTR) subjects (n=70) were enrolled for this study between 2008 and 2012. Twenty patients with primary biliary cirrhosis (PBC) were included as disease control group.

The study protocol was approved by the Institutional Review Board of Humanitas Clinical and Research Center, Rozzano (MI), Italy, and all subjects gave written informed consent.

### Study design

This study included two phases: 1) a discovery phase in which differentially expressed miRNAs were selected from profiling using high-throughput real-time PCR; and 2) an independent validation phase of miRNAs selected in the discovery phase. The samples were randomly included in the discovery or validation phase.

In the discovery phase inventors investigated the miRNAs expression profile in serum from 30 patients with CCA, 30 with PSC, and 30 CTR with similar gender and age (mean age 55 years, 60% male). Ten pools of three serum samples each (a total volume of 400 µl) were analyzed for each group; 667 miRNAs were evaluated in each pool. The aim of this phase was to obtain a specific profile of miRNA expression for each pathological condition.

In the validation phase all the miRNAs found to be differentially expressed in CCA and PSC were validated in an independent cohort of 120 individual serum samples (400 µl each), including 40 CTR, 40 PSC, and 40 CCA with similar gender and age. A group of 20 patients with PBC was used as disease control group of autoimmune cholestatic liver disease that does not predispose to the development of CCA.

### Extraction and quantification of circulating miRNAs

Peripheral blood samples were obtained and serum was isolated by centrifugation at 1700 g for 15 min within 2 hours after blood collection. Serum was recovered, dispensed into aliquots and stored at -80°C. Total RNA was extracted from serum samples using the mirVana Paris isolation kit (Ambion, Life Technologies) specific for liquid samples, and converted to cDNA by priming with stem-looped RT primers (Human Megaplex RT primers, Life Technologies) combined with the TaqMan® microRNA reverse transcription kit (Life Technologies). miRNA quantification was performed with the microfluidic card TaqMan® Array (Life Technologies).

### Primers from Life Technologies:

Gene Symbol: hsa-miR-483-5p
   Mature miRNA Sequence: AAGACGGGAGGAAAGAAGGGAG (SEQ ID NO:1)
   Assay ID: 002338
Gene Symbol: hsa-miR-194
   Mature miRNA Sequence: UGUAACAGCAACUCCAUGUGGA (SEQ ID NO:2)
   Assay ID: 000493
Gene Symbol: hsa-miR-222
   Mature miRNA Sequence: AGCUACAUCUGGCUACUGGGU (SEQ ID NO:3)
   Assay ID: 002276
Gene Symbol: hsa-miR-200c
   Mature miRNA Sequence: UAAUACUGCCGGGUAAUGAUGGA (SEQ ID NO:4)
   Assay ID: 002300
Gene Symbol: hsa-miR-193-b
   Mature miRNA Sequence: AACUGGCCCUCAAAGUCCCGCU (SEQ ID NO:5)
   Assay ID: 002367
Gene Symbol: hsa-miR-122
   Mature miRNA Sequence: UGGAGUGUGACAAUGGUGUUUG (SEQ ID NO:6)
   Assay ID: 002245
Gene Symbol: hsa-miR-885-5p
   Mature miRNA Sequence: UCCAUUACACUACCCUGCCUCU (SEQ ID NO:7)
   Assay ID: 002296

### Results

### Discovery phase: identification of disease-associated miRNA candidates

In this study inventors analyzed the miRNA profiling results with the aim to identify circulating miRNAs specific for PSC and CCA. A total of 667 miRNAs were evaluated in 10 pools of 3 subjects for each group. An average 219 out of 667 miRNAs were detected per sample (Figure 1).

Inventors performed the following comparisons to obtain miRNAs signatures: a) CCA and PSC samples compared to CTR samples to identify disease correlated candidate miRNAs deregulated in PSC and CCA; b) PSC compared to CTR samples to identify PSC-associated candidate miRNAs; c) CCA compared to CTR samples to extract CCA-associated candidate miRNAs, and d) CCA compared to PSC samples.

Using the criteria illustrated in the statistical analysis section (Figure 2), inventors obtained a list of candidate miRNAs (Figure 3) which were carried forward to the validation phase.

### Validation phase: disease-associated miRNAs

Serum miRNAs identified in the discovery phase were validated using an independent cohort of PSC (n=40), CCA (n=40), and CTR subjects (n=40). As stated before, inventors used single subject serum sample for validation.

Similarly to the discovery phase, inventors compared a) CCA and PSC (combined) to CTR samples b) PSC to CTR samples; c) CCA to CTR samples, and d) CCA to PSC

Using the criteria stated in the statistical analysis section, inventors found that miR-200c was specifically down-regulated in PSC (Figure 4) whereas miR-483-5p and miR-194 were upregulated in CCA (Figure 5) when compared to CTR. Importantly, inventors also found that miR-222 and miR-483-5p were specifically upregulated in CCA when compared to PSC (Figure 6). The combined ROC analysis of identified miRNAs from each disease group significantly increases the AUC value, suggesting that the combination of these markers makes the diagnosis even more specific and reliable.

Inventors also identified miR-193b, miR-122 and miR-885-5p upregulated in PSC and CCA compared to healthy control group (Figure 7); these miRNA have been reported to be altered in other liver diseases and can be considered not specific. These results were also confirmed by the analysis of PBC patients, an autoimmune cholestatic liver disease that does not predispose to the development of CCA, since their expression is also significantly different from the CTR group (Table 2).

**Table 2. Deregulated miRNAs analyzed in sera from PBC patients (10 early and 10 advanced) compared to CTR subjects**

| **miRNA** | **FC** | **p-value** |
|---|---|---|
| miR-122 | 10.46 | 0.012 |
| miR-885-5p | 7.09 | 0.005 |
| miR-193b | 2.11 | 0.036 |
| miR-200c | 0.21 | ns |
| miR-483-5p | 2.11 | ns |
| miR-194 | 1.43 | ns |
| miR-222 | 0.96 | ns |

| | | |
|---|---|---|
| Abbreviations: FC=Fold Change | | |

### Statistical analysis

For the discovery phase, Ct data were extracted using RQ Manager Software version 1.2.1 and Data Assist Software version 3.01, and imported into R. Quality control and statistical analysis were performed using the package HTqPC. ΔCt was calculated using the mean of all expressed miRNAs (Ct < 40) as reference, then Relative Quantities were calculated as RQ=2^-ΔCt. Association with disease was evaluated with a t-test on the ΔCt value. A miRNA was considered differentially represented if its absolute log2(fold change) > 1 and p-value < 0.05 (t-test between two populations). Only miRNAs that demonstrated an absolute log2(fold change) > 1, mean Ct < 30, and p < 0.05 were selected for validation.

For the validation phase, RQs were calculated using as reference the average Ct value of four endogenous controls (miR-17, miR-29a, miR-30c and miR-30b), identified by bioinformatic analysis (Figure 8). Inventors modelled the disease outcome as a function of the quantitative level of each miRNAs independently (Table 3). A standard t-test was performed on log10(RQ) and the difference was considered significant if p < 0.05. Odds ratio (OR) was obtained with logistic regression for each mRNA. For an alteration of a given miRNAs in log scale, inventors obtained an OR equal to the one indicated on each row and for each column (Table 4). The reference is the control group, or the PSC group when contrasting CCA compared to PSC. OR were filtered on the basis of the corresponding p-value, considered significant if p<0.05 (Table 5). For each miRNA, inventors fitted a logistic model and inventors extracted the coefficient (beta) relative to the effect of the miRNAs. This coefficient may be interpreted as the increase of log odds per unit increase of log10(RQ) of each miRNA. OR was calculated as OR = ebeta. The p-value associated to each OR was considered significant if p < 0.05. ROC curves were obtained using logistic regression, and the Area Under the Curve (AUC) was calculated using epicalc package (R package version 2.15.1.0.)

To evaluate the discriminating effect of serum miRNAs, ROC curve were established for each validated miRNAs. The AUC were calculated from the logistic regression (Table 6). The results revealed some miRNAs with large areas under the ROC curve (AUC>0.70) that are useful as biomarker.

**Table 3. miRNAs deregulated, evaluated in the validation phase and corresponding p-value.**

| | **p-value** | | | |
|---|---|---|---|---|
| **miRNA** | **CCAvsCTR** | **PSCvsCTR** | **CCAvsPSC** | **DISEASEvsCTR** |
| miR_122 | 0.000021 | 0.000319 | n.s. | 0.000014 |
| miR_125_5p | n.s. | n.s. | 0.022845 | n.s. |
| miR_125b | n.s. | n.s. | n.s. | n.s. |
| miR_132 | n.s. | 0.014411 | n.s. | 0.018953 |
| miR_138_1_star | n.s. | n.s. | n.s. | n.s. |
| miR_139_5p | 0.001024 | n.s. | 0.005352 | 0.029478 |
| miR_140_3p | n.s. | n.s. | 0.044674 | n.s. |
| miR_150 | n.s. | n.s. | n.s. | n.s. |
| miR_151_3p | n.s. | 0.022272 | 0.036024 | 0.048131 |
| miR_16 | n.s. | 0.001716 | 0.000451 | n.s. |
| miR_185 | n.s. | 0.004192 | 0.001292 | n.s. |
| miR_186 | n.s. | n.s. | n.s. | n.s. |
| miR_188_5p | n.s. | n.s. | n.s. | n.s. |
| miR_192 | n.s. | 0.015247 | n.s. | n.s. |
| miR_193b | 0.000023 | 0.034030 | n.s. | 0.000463 |
| miR_194 | 0.009867 | n.s. | n.s. | 0.027675 |
| miR_195 | n.s. | 0.000266 | 0.002296 | n.s. |
| miR_197 | n.s. | n.s. | n.s. | n.s. |
| miR_200c | n.s. | 0.000139 | 0.000030 | n.s. |
| miR_21 | n.s. | 0.004119 | n.s. | 0.005567 |
| miR_222 | n.s. | 0.042421 | 0.0011 | n.s. |
| miR_28_3p | n.s. | n.s. | n.s. | n.s. |
| miR_29c | n.s. | n.s. | 0.023531 | n.s. |
| miR_30a_3p | n.s. | n.s. | n.s. | n.s. |
| miR_30a_5p | n.s. | 0.003977 | n.s. | 0.026855 |
| miR_30d | n.s. | 0.001420 | n.s. | n.s. |
| miR_30e* | 0.015595 | n.s. | 0.005087 | n.s. |
| miR_320 | n.s. | n.s. | n.s. | n.s. |
| miR_342_3p | n.s. | n.s. | n.s. | n.s. |
| miR_345 | n.s. | n.s. | n.s. | n.s. |
| miR_34a_star | 0.016952 | n.s. | n.s. | n.s. |
| miR_375 | n.s. | n.s. | n.s. | n.s. |
| miR_454 | n.s. | 0.031205 | 0.017633 | n.s. |
| miR_483_5p | 0.000047 | n.s. | 0.001309 | 0.002540 |
| miR_625_star | n.s. | n.s. | n.s. | n.s. |
| miR_632 | n.s. | n.s. | n.s. | n.s. |
| miR_760 | 0.029292 | n.s. | 0.025910 | n.s. |
| miR_885_5p | 0.001266 | 0.007936 | n.s. | 0.000302 |
| miR_93_star | n.s. | n.s. | n.s. | n.s. |

**Table 4. Odds Ratio of each miRNA**

| | **Odds Ratio** | | | |
|---|---|---|---|---|
| **miRNA** | **CCAvsCTR** | **PSCvsCTR** | **CCAvsPSC** | **DISEASEvsCTR** |
| miR_122 | 7.598 | 4.155 | 1.231 | 4.665 |
| miR_125a_5p | 1.310 | 0.615 | 1.898 | 0.798 |
| miR_125b | 1.228 | 1.123 | 1.077 | 1.170 |
| miR_132 | 1.383 | 1.610 | 0.851 | 1.492 |
| miR_138_1_star | 1.144 | 1.086 | 1.025 | 1.109 |
| miR_139_5p | 18.036 | 1.325 | 8.874 | 4.234 |
| miR_140_3p | 0.473 | 1.399 | 0.409 | 0.877 |
| miR_150 | 0.423 | 0.549 | 0.819 | 0.512 |
| miR_151_3p | 0.843 | 0.547 | 1.734 | 0.644 |
| miR_16 | 0.958 | 10.627 | 0.054 | 3.091 |
| miR_185 | 1.046 | 3.081 | 0.257 | 1.431 |
| miR_186 | 1.548 | 0.967 | 1.454 | 1.21 |
| miR_188_5p | 0.949 | 1.141 | 0.864 | 1.027 |
| miR_192 | 1.252 | 2.270 | 0.526 | 1.698 |
| miR_193b | 7.517 | 2.207 | 1.415 | 3.193 |
| miR_194 | 1.832 | 1.298 | 1.327 | 1.449 |
| miR_195 | 0.794 | 22.443 | 0.047 | 1.922 |
| miR_197 | 0.889 | 0.764 | 0.940 | 0.883 |
| miR_200c | 1.407 | 0.346 | 2.306 | 0.808 |
| miR_21 | 3.190 | 8.286 | 0.859 | 3.274 |
| miR_222 | 4.622 | 0.219 | 10.505 | 0.850 |
| miR_28_3p | 0.885 | 0.372 | 1.217 | 0.734 |
| miR_29c | 0.828 | 1.717 | 0.416 | 1.173 |
| miR_30a_3p | 1.780 | 0.675 | 1.935 | 0.969 |
| miR_30a_5p | 1.934 | 13.778 | 0.365 | 3.566 |
| miR_30d | 1.209 | 14.930 | 0.254 | 2.328 |
| miR_30e* | 5.839 | 0.771 | 9.355 | 2.268 |
| miR_320 | 2.662 | 0.789 | 2.335 | 1.173 |
| miR_342_3p | 0.632 | 2.204 | 0.282 | 1.146 |
| miR_345 | 1.262 | 0.883 | 1.303 | 1.009 |
| miR_34a_star | 1.574 | 1.126 | 1.273 | 1.298 |
| miR_375 | 0.811 | 0.907 | 0.862 | 0.849 |
| miR_454 | 1.455 | 0.311 | 3.103 | 0.658 |
| miR_483_5p | 5.968 | 1.743 | 4.684 | 3.395 |
| miR_625_star | 1.991 | 1.795 | 1.131 | 2.005 |
| miR_632 | 0.950 | 1.070 | 0.903 | 1.003 |
| miR_760 | 1.487 | 0.980 | 1.487 | 1.213 |
| miR_885_5p | 4.231 | 2.964 | 1.347 | 2.870 |
| miR_93_star | 0.867 | 1.777 | 0.705 | 1.046 |

**Table 5. The p-value associated to each Odds Ratio.**

| | **p-value Odds Ratio** | | | |
|---|---|---|---|---|
| **miRNA** | **CCAvsCTR** | **PSCvsCTR** | **CCAvsPSC** | **DISEASEvsCTR** |
| miR_122 | 0.000194 | 0.001249 | n.s. | 0.000075 |
| miR_125a_5p | n.s. | n.s. | 0.036370 | n.s. |
| miR_125b | n.s. | n.s. | n.s. | n.s. |
| miR_132 | n.s. | 0.017109 | n.s. | 0.017494 |
| miR_138_1_star | n.s. | n.s. | n.s. | n.s. |
| miR_139_5p | 0.002896 | n.s. | 0.008668 | 0.046137 |
| miR_140_3p | n.s. | n.s. | n.s. | n.s. |
| miR_150 | n.s. | n.s. | n.s. | n.s. |
| miR_151_3p | n.s. | 0.033025 | 0.048523 | n.s. |
| miR_16 | n.s. | 0.003276 | 0.001277 | n.s. |
| miR_185 | n.s. | 0.027045 | 0.008062 | n.s. |
| miR_186 | n.s. | n.s. | n.s. | n.s. |
| miR_188_5p | n.s. | n.s. | n.s. | n.s. |
| miR_192 | n.s. | 0.019943 | n.s. | n.s. |
| miR_193b | 0.000176 | 0.041912 | n.s. | 0.002455 |
| miR_194 | 0.015660 | n.s. | n.s. | 0.037358 |
| miR_195 | n.s. | 0.000990 | 0.001532 | n.s. |
| miR_197 | n.s. | n.s. | n.s. | n.s. |
| miR_200c | n.s. | 0.001615 | 0.000446 | n.s. |
| miR_21 | n.s. | 0.006861 | n.s. | 0.045464 |
| miR_222 | n.s. | 0.048808 | 0.003125 | 0.782634 |
| miR_28_3p | n.s. | n.s. | n.s. | n.s. |
| miR_29c | n.s. | n.s. | 0.031349 | n.s. |
| miR_30a_3p | n.s. | n.s. | n.s. | n.s. |
| miR_30a_5p | n.s. | 0.006673 | n.s. | n.s. |
| miR_30d | n.s. | 0.003504 | n.s. | n.s. |
| miR_30e* | 0.023612 | n.s. | 0.009940 | n.s. |
| miR_320 | n.s. | n.s. | n.s. | n.s. |
| miR_342 3p | n.s. | n.s. | n.s. | n.s. |
| miR_345 | n.s. | n.s. | n.s. | n.s. |
| miR_34a_star | 0.020476 | n.s. | n.s. | n.s. |
| miR_375 | n.s. | n.s. | n.s. | n.s. |
| miR_454 | n.s. | 0.036442 | 0.022515 | n.s. |
| miR_483_5p | 0.000344 | n.s. | 0.003076 | 0.002720 |
| miR_625_star | n.s. | n.s. | n.s. | n.s. |
| miR_632 | n.s. | n.s. | n.s. | n.s. |
| miR_760 | 0.033680 | n.s. | 0.030031 | n.s. |
| miR_885_5p | 0.002109 | 0.011260 | n.s. | 0.002940 |
| miR_93_star | n.s. | n.s. | n.s. | n.s. |

**Table 6. Area under curve (AUC) of each miRNA**

| | **AUC** | | | |
|---|---|---|---|---|
| **miRNA** | **CCAvsCTR** | **PSCvsCTR** | **CCAvsPSC** | **DISEASEvsCTR** |
| miR_122 | 0.813 | 0.749 | 0.554 | 0.781 |
| miR_125a_5p | 0.512 | 0.578 | 0.583 | 0.532 |
| miR_125b | 0.585 | 0.544 | 0.526 | 0.564 |
| miR_132 | 0.604 | 0.637 | 0.553 | 0.620 |
| miR_138_1_star | 0.559 | 0.493 | 0.543 | 0.526 |
| miR_139_5p | 0.703 | 0.495 | 0.686 | 0.599 |
| miR_140_3p | 0.572 | 0.591 | 0.666 | 0.490 |
| miR_150 | 0.580 | 0.539 | 0.532 | 0.561 |
| miR_151_3p | 0.578 | 0.581 | 0.549 | 0.579 |
| miR_16 | 0.487 | 0.706 | 0.716 | 0.609 |
| miR_185 | 0.446 | 0.737 | 0.781 | 0.591 |
| miR_186 | 0.538 | 0.471 | 0.518 | 0.534 |
| miR_188_5p | 0.498 | 0.556 | 0.551 | 0.529 |
| miR_192 | 0.625 | 0.715 | 0.599 | 0.670 |
| miR_193b | 0.761 | 0.715 | 0.523 | 0.738 |
| miR_194 | 0.741 | 0.629 | 0.625 | 0.685 |
| miR_195 | 0.495 | 0.738 | 0.713 | 0.621 |
| miR_197 | 0.423 | 0.510 | 0.412 | 0.466 |
| miR_200c | 0.632 | 0.742 | 0.791 | 0.555 |
| miR_21 | 0.754 | 0.673 | 0.466 | 0.714 |
| miR_222 | 0.622 | 0.625 | 0.709 | 0.501 |
| miR_28_3p | 0.401 | 0.606 | 0.668 | 0.504 |
| miR_29c | 0.541 | 0.636 | 0.670 | 0.547 |
| miR_30a_3p | 0.658 | 0.501 | 0.621 | 0.421 |
| miR_30a_5p | 0.597 | 0.666 | 0.572 | 0.632 |
| miR_30d | 0.556 | 0.666 | 0.609 | 0.611 |
| miR_30e* | 0.684 | 0.483 | 0.653 | 0.600 |
| miR_320 | 0.583 | 0.502 | 0.583 | 0.538 |
| miR_342_3p | 0.540 | 0.596 | 0.630 | 0.527 |
| miR_345 | 0.609 | 0.464 | 0.580 | 0.572 |
| miR_34a_star | 0.645 | 0.483 | 0.611 | 0.564 |
| miR_375 | 0.623 | 0.597 | 0.547 | 0.610 |
| miR_454 | 0.528 | 0.601 | 0.636 | 0.536 |
| miR_483_5p | 0.766 | 0.615 | 0.700 | 0.700 |
| miR_625_star | 0.609 | 0.566 | 0.518 | 0.587 |
| miR_632 | 0.533 | 0.538 | 0.564 | 0.502 |
| miR_760 | 0.612 | 0.518 | 0.630 | 0.546 |
| miR_885_5p | 0.787 | 0.701 | 0.590 | 0.743 |
| miR_93_star | 0.485 | 0.512 | 0.500 | 0.512 |

In conclusion inventors confirmed the following disease-associated miRNAs: 1 PSC-specific miRNAs: the down-regulated miR-200c; 2 CCA- specific miRNAs, the upregulated miR-483-5p and miR-194; and 2 CCA-associated miRNA, miR-222 and miR-483-5p that are upregulated in comparison to PSC. Inventors also confirmed miR-193b, miR-122 and miR-885-5p as liver disease associated miRNAs upregulated in PSC and CCA compared to healthy control group The combined ROC analysis of identified miRNAs from each disease group significantly increases the AUC value, suggesting that the combination of these markers makes the diagnosis even more specific and reliable.

## Claims

1. An in vitro method for diagnosing and/or assessing the risk of developing and/or prognosing and/or for monitoring the progression and/or for monitoring the efficacy of a therapeutic treatment and/or for the screening of a therapeutic treatment of a disease of the bile ducts in a subject comprising the steps of:
a) detecting at least one microRNA selected from the group consisting of:
miR-483-5p, miR-194, miR-222 and miR-200c
in an isolated biological sample obtained from the subject and
b) comparing with respect to a proper control
wherein the disease of the bile ducts is selected from the group consisting of: Cholangiocarcinoma (CCA) and Primary sclerosing cholangitis (PSC).

2. An in vitro method for diagnosing and/or assessing the risk of developing and/or prognosing Cholangiocarcinoma (CCA) according to claim 1, comprising the steps of:
a) measuring the amount of miR-483-5p and/or miR-194 and/or miR-222
in said isolated biological sample obtained from the subject and
b) comparing the measured amount of step a) with a proper control amount,
wherein an amount of said miR-483-5p and/or miR-194 and/or miR-222 in the isolated biological sample obtained from the subject higher than the control amount indicates that the subject is either affected by or is at increased risk for developing CCA.

3. The in vitro method for diagnosing and/or assessing the risk of developing and/or prognosing Cholangiocarcinoma (CCA) according to claim 2, comprising the steps of:
a) measuring the amount of miR-483-5p and/or miR-194
in said isolated biological sample obtained from the subject and
b) comparing the measured amount of step a) with a proper control amount,
wherein an amount of said miR-483-5p and/or miR-194 in the isolated biological sample obtained from the subject higher than the control amount indicates that the subject is either affected by or is at increased risk for developing CCA.

4. An in vitro method for diagnosing and/or assessing the risk of developing and/or prognosing Primary sclerosing cholangitis (PSC) according to claim 1, comprising the steps of:
a) measuring the amount of miR-200c
in said isolated biological sample obtained from the subject and
b) comparing the measured amount of step a) with a proper control amount,
wherein an amount of said microRNA in the isolated biological sample obtained from the subject lower than the control amount indicates that the subject is either affected by or is at increased risk for developing PSC.

5. An in vitro method for diagnosing and/or assessing the risk of developing and/or prognosing CCA according to claim 1, comprising the steps of:
a) measuring the amount of miR-222 and/or miR-483-5p and/or miR-194
in said isolated biological sample obtained from the subject and
b) comparing the measured amount of step a) with a proper control amount from a PSC patient,
wherein an amount of said miR-222 and/or miR-483-5p and/or miR-194 in the isolated biological sample obtained from the subject higher than the control amount indicates that the subject is either affected by or is at increased risk for developing CCA.

6. The in vitro method for diagnosing and/or assessing the risk of developing and/or prognosing CCA according to claim 5, comprising the steps of:
a) measuring the amount of miR-222 and/or miR-483-5p
in said isolated biological sample obtained from the subject and
b) comparing the measured amount of step a) with a proper control amount from a PSC patient,
wherein an amount of said miR-222 and/or miR-483-5p in the isolated biological sample obtained from the subject higher than the control amount indicates that the subject is either affected by or is at increased risk for developing CCA.

7. An in vitro method for monitoring the progression and/or for monitoring the efficacy of a therapeutic treatment and/or for the screening of a therapeutic treatment of CCA or PSC according to claim 1, comprising the steps of:
a) measuring the alteration of the amount of at least one microRNA selected from the group consisting of:
miR-483-5p, miR-194, miR-222 and miR-200c
in said isolated biological sample obtained from the subject and
b) comparing the measured amount of step a) with a proper control amount.

8. The method according to any one of the preceding claims wherein the microRNAs to be detected or measured in step a) are:
- at least miR-483-5p and miR-194, or
- at least miR-222 and miR-483-5p, or
- at least miR-222 and miR-194, or
- at least miR-222, miR-483-5p and miR-194, or
- at least miR-200c.

9. The method according to any one of the previous claims wherein the biological sample is a blood sample, preferably a serum sample, a whole blood sample, or a plasma sample.

10. The method according to any one of the previous claims wherein said method further comprises the step of extracting RNA from the biological sample.

11. The method according to any one of the previous claims wherein the detection or the measure of the amount or of the alteration of the amount of the microRNA comprises specific acid nucleic amplification and/or hybridization, e.g. by RT-qPCR.

12. A kit for carrying out the method of any one of claims 1-11, comprising
- means to detect and/or measure the amount of the at least one microRNA as defined in any one of claims 1-8 and optionally,
- control means.

13. A kit to detect and/or measure the amount of the at least one microRNA as defined in any one of claims 1-8 consisting of:
- for each of said microRNA, sequence specific amplification means;
- quantitative detection means of said amplified nucleic acids;
- appropriate reagents.

14. A device for measuring the amount of at least one miRNA in a biological sample, wherein said device consists of:
- one or more probe sets for the miRNA as defined in any one of claims 1-8 and
- solid supporting means.

15. The device according to claim 14, wherein said device is a microarray chip, a QPCR Microfluidic Card, QPCR tubes, QPCR tubes in a strip, or a QPCR plate.
